(19) Europäisches Patentamt
European Patent Office
Office européen des brevets

(11) Veröffentlichungsnummer: **0 373 437 A2**

# (12) EUROPÄISCHE PATENTANMELDUNG

(21) Anmeldenummer: **89122176.4**

(51) Int. Cl.5: **C07D 401/14, C08K 5/3435**

(22) Anmeldetag: **01.12.89**

Patentansprüche für folgenden Vertragsstaat: ES.

(30) Priorität: **16.12.88 DE 3842304**

(43) Veröffentlichungstag der Anmeldung: **20.06.90 Patentblatt 90/25**

(84) Benannte Vertragsstaaten: **BE CH DE ES FR GB IT LI NL**

(71) Anmelder: **BASF Aktiengesellschaft**
**Carl-Bosch-Strasse 38**
**D-6700 Ludwigshafen(DE)**

(72) Erfinder: **Aumueller, Alexander, Dr.**
**An der Marlach 5**
**D-6705 Deidesheim(DE)**
Erfinder: **Neumann, Peter, Dr.**
**Poststrasse 28**
**D-6800 Mannheim 31(DE)**
Erfinder: **Trauth, Hubert**
**Milanstrasse 5**
**D-6724 Dudenhofen(DE)**

## (54) Polyalkylpiperidinsubstituierte Lactame und deren Verwendung als Stabilisatoren für Kunststoffe.

(57) Verbindungen der Formel (I)

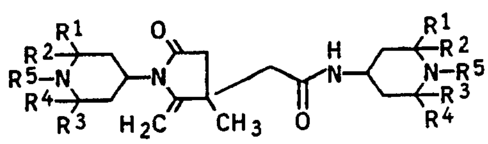 (I),

in der $R^1$, $R^2$, $R^3$ und $R^4$ für Alkyl oder $R^1$ und $R^2$ oder $R^3$ und $R^4$ eine Tetra-oder Pentamethylengruppierung und R5 für Wasserstoff, Alkanoyl, Alkyl, Cyanalkyl, Hydroxyalkyl, Aminoalkyl oder Phenylalkyl stehen, wobei der Phenylkern gegebenenfalls durch $C_1$-$C_4$-Alkyl, $C_1$-$C_4$-Alkoxy oder Halogen substituiert ist, darstellen, sowie die Säureadditionssalze und Hydrate dieser Verbindungen.
Die Verbindungen (I) sind Stabilisatoren für organische Materialien, insbesondere für Kunststoffe.

EP 0 373 437 A2

## Polyalkylpiperidinsubstituierte Lactame und deren Verwendung als Stabilisatoren für Kunststoffe

Es ist bekannt, daß Polyalkylpiperidinderivate organische Polymere vor Zerstörung durch Licht und Wärme schützen.

Unbefriedigend ist bei den Stabilisatoren des Standes der Technik oft deren Verträglichkeit mit Polyolefinen und anderen Kunststoffen, die Dauer der Schutzwirkung, die Eigenfarbe der Substanzen, deren Flüchtigkeit und deren thermische Zersetzung beim Einarbeiten in die Polymeren bei erhöhter Temperatur.

Der Erfindung liegt die Aufgabe zugrunde, neue Stabilisatoren zur Verfügung zu stellen, welche die vorstehenden Nachteile nicht aufweisen.

Die Aufgabe wird mit Hilfe der neuen Lactame der Formel (I) gelöst. Dementsprechend betrifft die Erfindung Polyalkylpiperidin-substituierte Lactame der allgemeinen Formel (I)

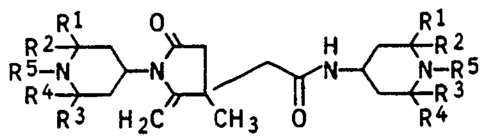 (I),

in der $R^1$, $R^2$, $R^3$ und $R^4$ für $C_1$-$C_4$-Alkyl oder $R^1$ und $R^2$ oder $R^3$ und $R^4$ eine Tetra- oder Pentamethylengruppierung und
$R^5$ für Wasserstoff, $C_1$-$C_8$-Alkanoyl, $C_1$-$C_8$-Alkyl,$C_1$-$C_3$-Cyanalkyl, $C_2$-$C_4$-Hydroxyalkyl, $C_2$-$C_3$-Aminoalkyl oder für $C_7$-$C_{10}$-Phenylalkyl stehen, wobei der Phenylkern unsubstituiert oder durch ein oder zwei $C_1$-$C_4$-Alkyl, $C_1$-$C_4$-Alkoxy oder Halogen substituiert ist,
sowie die Säureadditionssalze und Hydrate dieser Verbindungen.

Die Verbindungen (I) eignen sich sehr gut zum Stabilisieren von organischem Material, speziell von Kunststoffen gegen den Abbau durch Licht und Wärme. Sie sind auch wirksam als Metalldesaktivator. Bevorzugt sind die neuen Verbindungen zum Stabilisieren von Polyolefinen, insbesondere von Polyethylen und Polypropylen, sowie von Polyurethanen und ABS.

Den zu stabilisierenden Kunststoffen werden die Verbindungen (1) in einer Konzentration von 0,01 bis 5 Gew.%, vorzugsweise 0,02 bis 2 Gew.% - bezogen auf das Polymere - vor, während oder nach der Polymerbildung zugesetzt.

Alkylgruppen für $R^1$ bis $R^4$ sind beispielsweise Methyl, Ethyl, n-Propyl, iso-Propyl, n-Butyl oder iso-Butyl. Zwei benachbarte Reste $R^1$ und $R^2$ oder $R^3$ und $R^4$ können zusammen auch eine Tetra- oder Pentamethylengruppierung darstellen.

Vorzugsweise stehen $R^1$ bis $R^4$ für Methyl.

Die Alkylreste $R^5$ können linear oder verzweigt sein. im einzelnen sind z.B. zu nennen: Methyl, Ethyl, Propyl, n-Butyl, n-Pentyl, iso-Pentyl, n-Hexyl, n-Heptyl oder n-Octyl. Bevorzugter Alkylrest für $R^5$ ist Methyl.

Alkanoyl für $R^5$ sind z.B. Propionyl, Butanoyl, Pentanoyl, Hexanoyl, Heptanoyl, Octanoyl, Benzoyl und insbesondere Acetyl. Phenylalkylreste für $R^5$ sind beispielsweise Phenylethyl und vorzugsweise Benzyl.

Als im Phenylkern substituierte Phenylalkylreste kommen z.B. 3- oder 4-Methoxybenzyl, 3- oder 4-Methoxyphenylethyl, 3- oder 4-Chlorbenzyl, 3-oder 4-Chlorphenylethyl, 3- oder 4-Ethoxybenzyl, 3- oder 4-Ethoxyphenylethyl und vorzugsweise 3- oder 4-Methylbenzyl in Betracht. Cyanalkylreste für $R^5$ sind beispielsweise Cyanethyl und vorzugsweise Cyanmethyl.

Hydroxyalkylreste für $R^5$ sind beispielsweise 3-Hydroxypropyl, 4-Hydroxybutyl, vorzugsweise 2-Hydroxyethyl. Aminoalkylgruppen für $R^5$ sind beispielsweise 3-Aminopropyl und vorzugsweise 2-Aminoethyl. Besonders bevorzugt für $R^5$ ist Wasserstoff.

Die erfindungsgemäßen Verbindungen lassen sich durch Aminolyse der Verbindung der Formel (II) mit Aminen der allgemeinen Formel (III) mit oder ohne Lösungsmittel gegebenenfalls in Gegenwart eines Katalysators herstellen. Bevorzugt arbeitet man ohne Lösungsmittel und ohne Katalysator mit einem Überschuß an Amin bei erhöhten Temperaturen zwischen 100 bis 220 °C, bevorzugt bei 110 bis 160 °C.

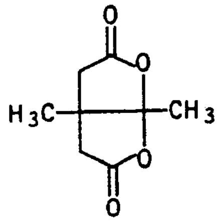

(II)

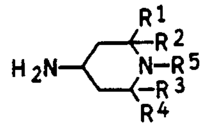

(III)

Verbindungen der allgemeinen Formel (I) mit $R^5$ gleich Wasserstoff lassen sich nach an sich literaturbekannten Verfahren wie Alkylierung, Acylierung, Michaeladdition, Cyanmethylierung oder Hydroxyalkylierung in Verbindungen der allgemeinen Formel (I) mit $R^5$ ungleich Wasserstoff umwandeln.

Die erfindungsgemäßen Verbindungen (I) können in Form der freien Basen, als Hydrate oder als Salze vorliegen. Geeignete Anionen stammen z. B. von anorganischen Säuren, Carbonsäuren und Sulfonsäuren. Von den Salzen sind die der Carbonsäuren und Sulfonsäuren bevorzugt.

Als anorganische Anionen kommen z. B. Chlorid, Bromid, Sulfat, Methosulfat, Tetrafluoroborat, Phosphat und Rhodanid in Betracht.

Carbonsäure-Anionen sind beispielsweise: Formiat, Acetat, Propionat, Hexanoat, Cyclohexanoat, Lactat, Stearat, Palmitat, Dodecylbenzoat, Benzoat, Acrylat, Methacrylat, Zitrat, Malonat oder Succinat sowie Anionen von Polycarbonsäuren, wie Polyacrylsäure, Polymethacrylsäure und (Meth)-Acrylsäure enthaltenden Copolymeren mit bis zu 3000 COOH-Gruppen.

Sulfonsäure-Anionen sind beispielsweise Benzolsulfonat, Tosylat und Methylsulfonat.

Die Erfindung betrifft außerdem die Verwendung von (I) als Stabilisator für organisches Material.

Das Einarbeiten der Verbindungen (I) in die zu stabilisierenden Kunststoffe kann in allen bekannten Vorrichtungen nach den bekannten Verfahren zum Einmischen von Stabilisierungsmitteln oder anderen Zusätzen in Polymere erfolgen.

Neben den erfindungsgemäßen Verbindungen (I) können die stabilisierten Kunststoffe gegebenenfalls noch weitere Additive enthalten, beispielsweise Antioxidantien, zusätzliche Lichtstabilisierungsmittel, Metalldesaktivatoren, antistatische Mittel, flammhemmende Mittel, außerdem Pigmente und Füllstoffe.

Antioxidantien und Lichtstabilisatoren, die den Kunststoffen neben den erfindungsgemäßen Verbindungen zugesetzt werden können, sind z.B. Verbindungen auf der Basis sterisch gehinderter Phenole, Schwefel und/oder Phosphor enthaltende Costabilisatoren.

Als derartige phenolische Antioxidationsmittel seien beispielsweise 2,6-Di-tert.-butyl-4-methylphenol, n-Octadecyl-ß-(3,5-di-tert.-butyl-4-hydroxyphenyl)-propionat, 1,1,3-Tris-(2-methyl-4-hydroxy-5-tert.-butylphenyl)-butan, 1,3,5-Trimethyl-2,4,6-tris-(3,5-di-tert.-butyl-4-hydroxybenzyl)-benzol, 1,3,5-Tris-(3,5-di-tert.-butyl-4-hydroxybenzyl)-iso-cyanurat, 1,3,5-Tris-[ß-(3,5-di-tert.-butyl-4-hydroxyphenyl)-propionyloxyethyl]-isocyanurat, 1,3,5-Tris-(2,6,di-methyl-3-hydroxy-4-tert.-butylbenzyl)-isocyanurat und Pentaerythrit-tetrakis-[ß-(3,5-di-tert.-butyl-4-hydroxy-phenyl)-propionat] erwähnt.

Als phosphorhaltige Antioxidantien kommen z. B. Tris-(nonyl-phenyl)-phosphit, Distearylpentaerythritdiphosphit, Tris-(2,4-di-tert.-butylphenyl)-phosphit, Tris-(2-tert.-butyl-4-methylphenyl)-phosphit, Bis-(2,4-ditert.-butylphenyl)-pentaerythritdiphosphit und Tetrakis-(2,4-di-tert.-butylphenyl)-4,4′-biphenylendiphosphit in Betracht.

Weitere Antioxidantien und Lichtstabilisatoren, die zusammen mit den erfindungsgemäßen Verbindungen verwendet werden können, sind z.B. 2-(2′-Hydroxyphenyl)-benztriazole, 2-Hydroxybenzophenone, Phenylester von Hydroxybenzoesäuren, α-Cyanozimtsäurederivate, Nickelverbindungen, Benzimidazolcarbonsäureanilide und/oder Oxalsäuredianilide.

Als organische Polymere, die durch die erfindungsgemäßen Verbindungen stabilisiert werden können, kommen z. B. in Betracht:

Polymere von Mono- und Diolefinen, wie Polyethylen niedriger und hoher Dichte, lineares Polyethylen niedriger Dichte, Polypropylen, Polyisobutylen, Polybuten-1, Polyisopren, Polybutadien sowie Copolymerisate von Mono- oder Diolefinen und Mischungen der genannten Polymeren;

Copolymerisate von Mono- oder Diolefinen mit anderen Vinylmonomeren z. B. Ethylen-Alkylacrylat-Copolymere, Ethylen-Alkylmethacrylat-Copolymere, Ethylen-Vinylacetat-Copolymere oder Ethylen-Acrylsäure-Copolymere;

Polystyrol;

Copolymere von Styrol oder α-Methylstyrol mit Dienen oder Acrylderivaten, wie Styrol-Butadien, Styrol-Acrylnitril, Styrol-Ethylmethacrylat, Styrol-Butadien-Ethylacrylat, Styrol-Acrylnitril-Methacrylat;

ABS-, MBS- oder ähnliche Polymere;
Halogenhaltige Polymere, z. B. Polyvinylchlorid, Polyvinylfluorid, Polyvinylidenfluorid sowie deren Copolymere;
Polymere die sich von α,ß-ungesättigten Säuren und deren Derivaten ableiten, wie Polyacrylate und Polymethacrylate, Polyacrylamide und Polyacrylnitrile;
Polymere, die sich von ungesättigten Alkoholen und Aminen bzw. deren Acrylderivaten oder Acetalen ableiten, wie Polyvinylalkohol oder Polyvinylacetat;
Polyurethane, Polyamide, Polyharnstoffe, Polyester, Polycarbonate, Polysulfone, Polyethersulfone und Polyetherketone.

Weiterhin können mit den Verbindungen (I) Lacküberzüge stabilisiert werden, z.B. Industrielackierungen. Unter diesen sind Einbrennlackierungen, von diesen wiederum Fahrzeuglackierungen, insbesondere Zweischichtlackierungen, besonders hervorzuheben. Auch hier können die bereits genannten weiteren Antioxidantien und Lichtschutzmittel noch zugesetzt werden.

Die erfindungsgemäßen Verbindungen können dem Lack in fester oder gelöster Form zugegeben werden. Die gute Löslichkeit von (I) in den Lacksystemen ist dabei von besonderem Vorteil.

Die erfindungsgemäßen Verbindungen sind vor allem zur Stabilisierung von Polyolefinen, insbesondere von Ethylen- und Propylenpolymerisaten, von Polyurethanen und Lacken geeignet.

Beispiel

14,5 g 1,5-Dimethyl-2,8-dioxa-cis-bicyclo[3.3.0]octa-3,7-dion (Herstellung siehe Chem. Ber. 108, 3256 (1975)) wurden in 60 g 4-Amino-2,2,6,6-tetramethylpiperidin 14 h auf 120 °C und anschließend 10 h auf 150 °C erhitzt. Nach dem Abkühlen wurden 250 ml Diethylether zugegeben, der ausgefallene Niederschlag wurde abgesaugt, mit Diethylether und Petrolether gewaschen und getrocknet. Ausbeute: 24,0 g der Verbindung der Formel

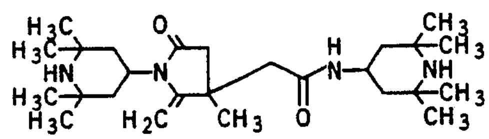

als farbloser Feststoff vom Schmp. 257 °C.

| | | | | |
|---|---|---|---|---|
| Ber.: | C 69,6 | H 10,8 | N 12,5 | O 7,1 % |
| Gef.: | C 69,7 | H 10,4 | N 12,5 | O 7,3 % |

Anwendungsbeispiel

Stabilisierung von Polypropylen

a) 0,25 Teile der Verbindung des Herstellbeispiels werden in 100 Teile Polypropylen (1320 H der Fa. BASF) durch zweimaliges Extrudieren bei 220 °C eingearbeitet und zu 200 µm dicken Platten gepreßt. Nach l4tägiger Lagerung im Dunkeln bei 25 °C zeigt die Oberfläche der Platte keinen Belag.

b) Die nach a) hergestellten Platten werden in einem Xenotest-1200-Schnellbewitterungs-Prüfgerät auf ihre Bewitterungsstabilität getestet. Die Alterung wird durch Messen der "CO-Zahl" nach bestimmten Zeitintervallen bestimmt. Der Beginn der Versprödung wird mechanisch ermittelt. Die Testergebnisse sind in der Tabelle zusammengefaßt.

Tabelle

| CO-Zahlen bei Belichtung in einem Xenotest-1200-Schnellbewitterungs-Prüfgerät (Polypropylen) | | | |
|---|---|---|---|
| zugesetzte Verbindung | Belichtungszeit in (h) | | |
| | 2000 | 3000 | 4000 |
| 1. Herstellbeispiel | 9,5 | 18,0 | 22,0 |
| 2. ohne | spröde | | |

## Ansprüche

1. Polyalkylpiperidinsubstituierte Lactame der allgemeinen Formel (I)

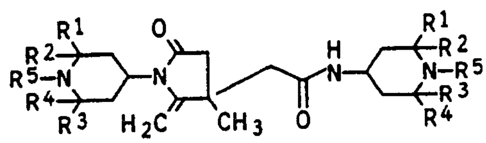 (I),

in der
$R^1$, $R^2$, $R^3$ und $R^4$ für $C_1$-$C_4$-Alkyl oder $R^1$ und $R^2$ oder $R^3$ und $R^4$ eine Tetra-oder Pentamethylengruppierung und
$R^5$ für Wasserstoff, $C_1$-$C_8$-Alkanoyl, $C_1$-$C_8$-Alkyl, $C_1$-$C_3$-Cyanalkyl, $C_2$-$C_4$-Hydroxyalkyl, $C_2$-$C_3$-Aminoalkyl oder für $C_7$-$C_{10}$-Phenylalkyl stehen, wobei der Phenylkern unsubstituiert oder durch ein oder zwei $C_1$-$C_4$-Alkyl, $C_1$-$C_4$-Alkoxy oder Halogen substituiert ist, sowie die Säureadditionssalze und Hydrate dieser Verbindungen.

2. Lactame gemäß Anspruch 1, dadurch gekennzeichnet, daß $R^1$, $R^2$, $R^3$ und $R^4$ Methyl bedeuten.

3. Lactame gemäß Anspruch 1 oder 2, dadurch gekennzeichnet, daß $R^5$ Wasserstoff bedeutet.

4. Verwendung von Verbindungen gemäß den Ansprüchen 1, 2 oder 3 zum Stabilisieren von organischem Material, insbesondere von Kunststoffen.

5. Verwendung von Verbindungen gemäß den Ansprüchen 1, 2 oder 3 zum Stabilisieren von Polyurethanen, Polyolefinen, ABS oder von Polyamiden.

6. Verwendung von Verbindungen gemäß den Ansprüchen 1, 2 oder 3 zum Stabilisieren von Lacken.

7. Verwendung von Verbindungen gemäß den Ansprüchen 1, 2 oder 3 als Metallionendesaktivator.

8. Stabilisiertes organisches Material, enthaltend Verbindungen gemäß den Ansprüchen 1, 2 oder 3.

Patentansprüche für folgenden Vertragsstaat: ES

1. Verwendung von Polyalkylpiperidin-substituierten Lactamen der allgemeinen Formel (I)

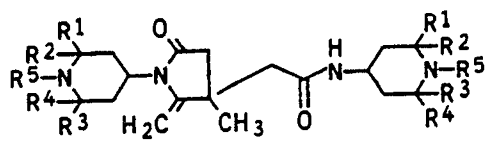 (I),

in der
$R^1$, $R^2$, $R^3$ und $R^4$ für $C_1$-$C_4$-Alkyl oder $R^1$ und $R^2$ oder $R^3$ und $R^4$ eine Tetra-oder Pentamethylengruppierung und
$R^5$ für Wasserstoff, $C_1$-$C_8$-Alkanoyl, $C_1$-$C_8$-Alkyl, $C_1$-$C_3$-Cyanalkyl, $C_2$-$C_4$-Hydroxyalkyl, $C_2$-$C_3$-Aminoalkyl oder für $C_7$-$C_{10}$-Phenylalkyl stehen, wobei der Phenylkern unsubstituiert oder durch ein oder zwei $C_1$-$C_4$-

Alkyl, $C_1$-$C_4$-Alkoxy oder Halogen substituiert ist, sowie die Säureadditionssalze und Hydrate dieser Verbindungen zum Stabilisieren von organischem Material.

2. Verwendung von Lactamen gemäß Anspruch 1, dadurch gekennzeichnet, daß in der Formel $R^1$, $R^2$, $R^3$ und $R^4$ Methyl bedeuten.

3. Verwendung von Lactamen gemäß Anspruch 1 oder 2, dadurch gekennzeichnet, daß in der Formel $R^5$ Wasserstoff bedeutet.

4. Verwendung von Polyalkylpiperidin-substituierten Lactamen der allgemeinen Formel (I)

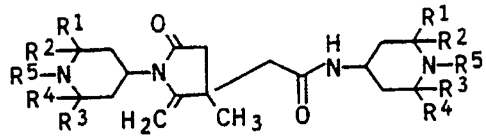

(I),

in der

$R^1$, $R^2$, $R^3$ und $R^4$ für $C_1$-$C_4$-Alkyl oder $R^1$ und $R^2$ oder $R^3$ und $R^4$ eine Tetra- oder Pentamethylengruppierung und

$R^5$ für Wasserstoff, $C_1$-$C_8$-Alkanoyl, $C_1$-$C_8$-Alkyl, $C_1$-$C_3$-Cyanalkyl, $C_2$-$C_4$-Hydroxyalkyl, $C_2$-$C_3$-Aminoalkyl oder für $C_7$-$C_{10}$-Phenylalkyl stehen, wobei der Phenylkern unsubstituiert oder durch ein oder zwei $C_1$-$C_4$-Alkyl, $C_1$-$C_4$-Alkoxy oder Halogen substituiert ist,

sowie die Säureadditionssalze und Hydrate dieser Verbindungen als Metallionendesaktivator.

5. Stabilisiertes organisches Material, enthaltend Polyalkylpiperidin-substituierte Lactame der allgemeinen Formel (I)

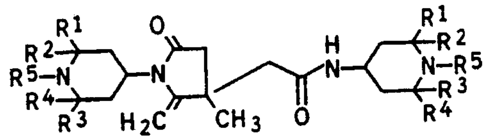

(I),

in der

$R^1$, $R^2$, $R^3$ und $R^4$ für $C_1$-$C_4$-Alkyl oder $R^1$ und $R^2$ oder $R^3$ und $R^4$ eine Tetra-oder Pentamethylengruppierung und

$R^5$ für Wasserstoff, $C_1$-$C_8$-Alkanoyl, $C_1$-$C_8$-Alkyl, $C_1$-$C_3$-Cyanalkyl, $C_2$-$C_4$-Hydroxyalkyl, $C_2$-$C_3$-Aminoalkyl oder für $C_7$-$C_{10}$-Phenylalkyl stehen, wobei der Phenylkern unsubstituiert oder durch ein oder zwei $C_1$-$C_4$-Alkyl, $C_1$-$C_4$-Alkoxy oder Halogen substituiert ist,

sowie die Säureadditionssalze und Hydrate dieser Verbindungen.